(19) Europäisches Patentamt
European Patent Office
Office européen des brevets

(11) Veröffentlichungsnummer: **0 119 428**
**A2**

(12) # EUROPÄISCHE PATENTANMELDUNG

(21) Anmeldenummer: **84101205.7**

(22) Anmeldetag: **07.02.84**

(51) Int. Cl.³: **C 07 C 103/30**, C 07 C 149/23, C 07 D 213/75, C 07 D 401/12, C 07 D 403/12, C 07 D 405/12, A 61 K 31/16, A 61 K 31/44

(30) Priorität: **18.02.83 DE 3305569**

(43) Veröffentlichungstag der Anmeldung: **26.09.84**
**Patentblatt 84/39**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Rosentreter, Ulrich, Dr., Kondorweg 23, D-5600 Wuppertal 1 (DE)**
Erfinder: **Niemers, Ekkehard, Dr., In den Birken 51a, D-5600 Wuppertal 1 (DE)**
Erfinder: **Stegelmeier, Hartmut, Dr., Melde la, D-4010 Hilden (DE)**
Erfinder: **Knorr, Andreas, Dr., Pahlkestrasse 15, D-5600 Wuppertal 1 (DE)**
Erfinder: **Garthoff, Bernward, Dr., Haendelstrasse 22, D-4010 Hilden (DE)**

(54) **Biscarboxamide zur Bekämpfung von Erkrankungen sowie Verfahren zu ihrer Herstellung.**

(57) Bis- (carboxamid)-Verbindungen der Formel

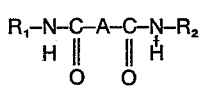 (I)

zur Verwendung bei der Bekämpfung von Erkrankungen, mehrere Verfahren zu ihrer Herstellung gemäß Variante A

$$HO\text{-}CO\text{-}A\text{-}COOR_4 \rightarrow R_3\text{-}CO\text{-}A\text{-}COOR_4$$

$$R_3\text{-}CO\text{-}A\text{-}COOR_4 + R_2\text{-}NH_2 \rightarrow R_2\text{-}NH\text{-}CO\text{-}A\text{-}COOR_4$$

$$R_2\text{-}NH\text{-}CO\text{-}A\text{-}COOR_4 \rightarrow R_2\text{-}NH\text{-}CO\text{-}A\text{-}COOH$$

$$R_2\text{-}NH\text{-}CO\text{-}A\text{-}COOH \rightarrow R_2\text{-}NH\text{-}CO\text{-}A\text{-}CO\text{-}R_3$$

$$R_2\text{-}NH\text{-}CO\text{-}A\text{-}CO\text{-}R_3 + R_1\text{-}NH_2 \rightarrow R_2\text{-}NH\text{-}CO\text{-}A\text{-}CO\text{-}NHR_1$$

oder Variante B

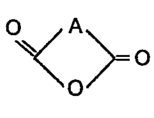 + $R_2\text{–}NH_2 \rightarrow R_2\text{–}NH\text{–}CO\text{–}A\text{–}COOH$

$$R_2\text{-}NH\text{-}CO\text{-}A\text{-}COOH \rightarrow R_2\text{-}NH\text{-}CO\text{-}A\text{-}CO\text{-}R_3$$

$$R_2\text{-}NH\text{-}CO\text{-}A\text{-}CO\text{-}R_3 + R_1\text{-}NH_2 \rightarrow R_2\text{-}NH\text{-}CO\text{-}A\text{-}CO\text{-}NH\text{-}R_1$$

sowie Arzneimittel enthaltende Bis-(carboxamid)-verbindungen als Wirkstoff.

EP 0 119 428 A2

BAYER AKTIENGESELLSCHAFT
5090 Leverkusen, Bayerwerk

Konzernverwaltung RP
Patentabteilung

E/ABc

# Biscarboxamide zur Bekämpfung von Erkrankungen sowie Verfahren zu ihrer Herstellung

Die Erfindung betrifft Bis-(carboxamid)-verbindungen der Formel I

$$R_1-\underset{H}{N}-\underset{\overset{\|}{O}}{C}-A-\underset{\overset{\|}{O}}{C}-\underset{H}{N}-R_2 \qquad (I)$$

zur Verwendung bei der Bekämpfung von Erkrankungen, mehrere Verfahren zu ihrer Herstellung, einige neue Verbindungen der Formel I, sowie Arzneimittel enthaltend als Wirkstoff Verbindungen der Formel I.

In der Formel I bedeuten

$R_1$ einen Arylrest mit bevorzugt 6-10 C-Atomen, wie beispielsweise Phenyl oder Naphthyl, der gegebenenfalls, bevorzugt 1-3 mal, substituiert ist durch gleiche oder verschiedene Substituenten aus der Gruppe Halogen (z.B. Fluor, Chlor, Brom), $C_1$-$C_4$-Alkyl, $C_1$-$C_4$-Alkoxy, $C_1$-$C_4$-Alkylmercapto, jeweils $C_1$-$C_4$-Dialkylamino, $C_1$-$C_4$-Mono-

 

alkylamino, Hydroxy, Mercapto, Amino, Nitro, Cyano, Trifluormethyl, oder einen Heteroarylrest mit 5-6 Ringatomen, wobei 1 oder 2 Atome Stickstoff, Sauerstoff oder Schwefel sind, bevorzugt aus der Gruppe Furyl, Thiophenyl, Pyrryl, Imidazyl, Pyridyl, Pyrimidyl, der gegebenenfalls einfach substituiert ist durch Substituenten aus der Gruppe Haloge (z.B. Fluor, Chlor, Brom), Trifluormethyl, Nitro, Cyano, $C_1$-$C_4$-Alkyl, $C_1$-$C_4$-Alkoxy, $C_1$-$C_4$-Alkylmercapto,

$R_2$ einen verzweigten Alkylrest mit bevorzugt 4-10 C-Atomen oder einen Cycloalkylrest mit bevorzugt 6-10 C-Atomen,

A eine Einfachbindung, einen Alkylenrest mit bevorzugt 1-5 C-Atomen, einen Alkenylrest mit bevorzugt 2-6 C-Atomen, eine Gruppe der Formel $-CH_2-S-CH_2-$ oder $-CH_2-O-CH_2-$, einen bivalenten Cycloalkylenrest mit bevorzugt 4-6 C-Atomen, einen bivalenten Cycloalkenylrest mit bevorzugt 4-6 C-Atomen, einen bivalenten Cycloalkenylrest mit bevorzugt 6-7 C-Atomen, einen Arylenrest mit bevorzugt 6-10 C-Atomen, einen Aralkylenrest mit bevorzugt 8-10 C-Atomen, einen bivalenten Heteroalkylrest mit 5-6 Ringatomen, wobei 1 oder 2 Atome Stickstoff, Sauerstoff oder Schwefel sind, bevorzugt aus der Gruppe Furan, Thiophen, Imidazol, Pyrrol, Oxazol, Thiazol, Pyrazol, Pyrazin, Pyridin

Die Erfindung betrifft auch ihre physiologisch unbedenklichen Salze.

Diese Salze sind beispielsweise Hydrochloride, Hydrogensulfate, Sulfate, Hydrogenphosphate, Acetate, Maleate, Benzoate, Citronate, Tartrate oder Lactate.

Von besonderem Interesse sind Verbindungen der Formel I, in der

$R_1$ einen Phenylrest darstellt, der gegebenenfalls 1-3 mal substituiert ist durch gleiche oder verschiedene Substituenten aus der Gruppe Halogen (z.B. Fluor, Chlor, Brom), Trifluormethyl, Nitro, Cyano, Hydroxy, Amino, $C_1$-$C_4$-Alkyl, $C_1$-$C_4$-Alkoxy, $C_1$-$C_4$-Alkylmercapto, jeweils $C_1$-$C_4$-Dialkylamino, oder einen Heteroarylrest mit 5-6 Ringatomen darstellt, wobei 1 oder 2 Atome Stickstoff, Sauerstoff oder Schwefel sind, bevorzugt aus der gruppe Furyl, Thiophenyl, Pyridyl, Pyrimidyl, der gegebenenfalls einfach substituiert ist durch Substituenten aus der Gruppe Halogen (z. B. Fluor, Chlor, Brom), Trifluormethyl, Nitro, Cyano, $C_1$-$C_4$-Alkyl, $C_1$-$C_4$-Alkoxy, $C_1$-$C_4$-Alkylmercapto,

$R_2$ einen verzweigten $C_4$-$C_{10}$-Alkylrest oder einen $C_6$-$C_{10}$-Cycloalkylrest darstellt,

A eine Einfachbindung, einen $C_1$-$C_5$-Alkylenrest, einen $C_2$-$C_6$-Alkenylrest, einen bivalenten $C_4$-$C_6$-Cycloalkylenrest, eine Gruppe der Formel $-CH_2-S-CH_2-$ oder $-CH_2-O-CH_2-$, einen bivalenten $C_6$-$C_7$-Cycloalkenylrest, einen $C_6$-$C_{10}$-Arylenrest oder einen Heteroarylrest aus der Gruppe Furan, Thiophen, Imidazol, Oxazol, Thiazol, Pyrazol, Pyrazin oder Pyridin darstellt,

sowie ihre physiologisch unbedenklichen Salze.

Besonders hervorgehoben seien Verbindungen der Formel I, in der

$R_1$ einen Phenylrest darstellt, der gegebenenfalls 1-3 mal substituiert ist durch gleiche oder verschiedene Substituenten aus der Gruppe Halogen (z.B. Fluor, Chlor, Brom), Trifluormethyl, $C_1$-$C_4$-Alkyl, $C_1$-$C_4$-Alkoxy, $C_1$-$C_4$-Alkylmercapto, oder Pyridyl oder Pyrimidyl darstellt, welche gegebenenfalls einfach substituiert sind durch Substituenten aus der Gruppe Halogen (z. B. Fluro, Chlor), Trifluormethyl, $C_1$-$C_4$-Alkyl, $C_1$-$C_4$-Alkoxy, $C_1$-$C_4$-Alkylmercapto,

$R_2$ einen verzweigten $C_4$-$C_{10}$-Alkylrest darstellt,

A eine Einfachbindung, einen $C_2$-$C_4$-Alkylen oder Alkenylrest darstellt oder eine Gruppe der Formeln $-CH_2-S-CH_2$ oder $-CH_2-O-CH_2-$ bedeutet, oder eine Phenylengruppe oder einen bivalenten Heteroarylrest aus der Gruppe Furan, Thiophen, Imidazol, Pyrazin oder Pyridin darstellt,

sowie ihre physiologisch unbedenklichen Salze.

Insbesonders seien Verbindungen der Formel I genannt, in der

$R_1$ Phenyl, 4-Hydroxyphenyl, 2-Ethoxyphenyl, Pydidyl, 6-Chlorpyridyl darstellt,

$R_2$ tert.-Butyl, 2,2-Dimethylpropyl, 1,2,2—Trimethylpropyl, 1,1,2,2-Tetramethylpropyl bedeutet,

A eine Einfachbindung, Methylen, Ethylen, Vinylen, Phenylen, die Gruppe $-CH_2-S-CH_2-$, einen bivalenten Heteroarylrest aus der Gruppe Furan, Thiophen, Pyrazin oder Pyridin darstellt,

sowie ihre pharmazeutisch unbedenklichen Salze.

Im einzelnen seien folgende Reste aufgeführt:

a) für $R_1$:

Phenyl, Naphthyl, Methylphenyl, n-Butylphenyl, Methoxyphenyl, Ethoxyphenyl, (n-Butoxy)phenyl, (Methylthio)phenyl, (n-Butylthio)phenyl, (Dimethylamino)phenyl, (Dibutylamino)phenyl, (Methylamino)phenyl, (Butylamino)phenyl, Chlorphenyl, Bromphenyl, Fluorphenyl, Chlor-(methyl)phenyl, Dimethylphenyl, (Hydroxy)-(methyl)phenyl, (Mercapto)-(methyl)phenyl, Trimethylphenyl, Chlormethoxyphenyl, Dichlorphenyl, Trichlorphenyl, Chlor-nitrophenyl, Methyl-nitrophenyl, Chlor-(trifluormethyl)phenyl, Di(trifluormethyl)phenyl, Ethyl-(methyl)phenyl, Dichlor-methoxyphenyl, Chlor-(methylthio)phenyl, (Hydroxy)-(methyl)phenyl, Chlor-hydroxyphenyl, Dimethoxyphenyl, Diethoxyphenyl, Chlor-dimethoxyphenyl,

b) für $R_2$:

tert.-Butyl, 1,1-Dimethylpropyl, 2,2-Dimethylpropyl, 1,2,2-Trimethylpropyl, 1,1-Dimethylbutyl, 1,1,2-Trimethylpropyl, 1-Ethyl-1-methylpropyl, 1,1,3-Trimethylbutyl, 1,1-Diethylpropyl, 1,1-Diethylbutyl, 1,1,2-Triethylbutyl, 1,1,2,2-Tetramethylpropyl, Cyclohexyl, Cyclooctyl, Adamantyl,

c) für A:

Methylen, Ethylen, Ethyliden, Propylen, Propyliden, Trimethylen, Isopropyliden, Butyliden, Ethylethylen, s-Methyltrimethylen, Tetramethylen, 1,2-Dimethylethylen oder2-Methyltrimethylethylen, Vinylen, Propenylen, Butenylen, Pentenylen, Butadienylen, Pentadienylen, Cyclohexan, Cyclopenten, Cyclobutan, Cyclohexen, Cyclohexadien, Cyclopenten, Cycloheptadien, Cycloheptatrien, Phenylen, Tolylen, Xylen, Naphthylen, Aralkylenreste der Formeln:

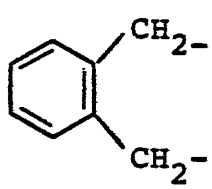 , 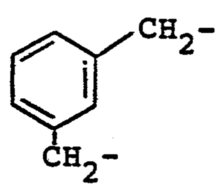 , 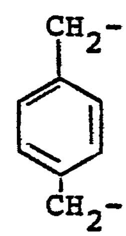 ,

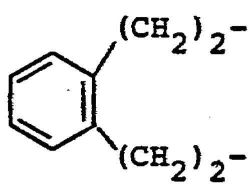 , 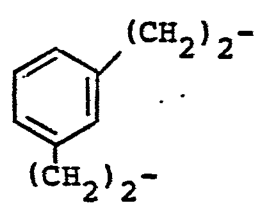 , 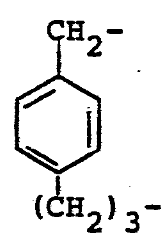 ,

Beispielhaft seien folgende Verbindungen genannt:

1. N-Phenyl-N'-(1,2,2-trimethylpropyl)-oxamid
2. N-(2-Ethoxyphenyl)-N'-(1,2,2-trimethylpropyl)-oxamid
3. N-Phenyl-N'-(1,2,2-trimethylpropyl)-fumaramid
4. N-(2-Ethoxyphenyl)-N'-(1,2,2-trimethylpropyl)-fumaramid
5. N-Phenyl-N'(2,3,3-trimethylpropyl)-succinamid
6. N-(2-Ethoxyphenyl)-N'-(1,2,2-trimethylpropyl)-succinamid
7. N-Phenyl-N'-(2,2-dimethylpropyl)-succinamid
8. N-Phenyl-N'-(1,2,2-trimethylpropyl)-thiodiglykolsäureamid
9. N-(2-Ethoxyphenyl)-N'-(1,2,2-trimethylpropyl)-thiodiglykolsäureamid
10. N-Phenyl-N'-(1,2,2-trimethylpropyl)-phtalamid
11. N-(2-Ethoxyphenyl)-N'-(1,2,2-trimethylpropyl)-phtalamid
12. N-(2-Ethoxyphenyl)-N'-(2,2-dimethylpropyl)-phthalamid
13. N-Phenyl-N'-(1,2,2-trimethylpropyl)-terephthalamid
14. N-(2-Ethoxy)-N'-(1,2,2-trimethylpropyl)-terephtalamid
15. N-(2-Ethoxy)-N'-(2,2-dimethylpropyl)-terephthalamid
16. N-Phenyl-N-(1,2,2-trimethylpropyl)-isophthalamid
17. N-(2-Ethoxyphenyl)-N'-(1,2,2-trimethylpropyl)-isophthalamid
18. N-(4-Hydroxyphenyl)-N'(1,2,2-trimethylpropyl)-phtalamid
19. N-(3-Pyridyl)-N'-(1,2,2-trimethylpropyl)-oxalamid
20. N-(4-Pyridyl)-N'-(1,2,2-trimethylpropyl)-oxalamid
21. N-(3-Pyridyl)-N'-(1,2,2-trimethylpropyl)-fumaramid
22. N-(3-Pyridyl)-N'-(1,2,2-trimethylpropyl)-succinamid

23. N-(4-Pyridyl)-N'-(1,2,2-trimethylpropyl)-succinamid
24. N-(3-Pyridyl)-N'-(1,2,2-trimethylpropyl)-thiodiglykolsäureamid
25. N-(4-Pyridyl)-N'-(1,2,2-trimethylpropyl)-thiodiglykolsäureamid
26. N-(3-Pyridyl)-N'-(1,2,2-trimethylpropyl)-phtalamid
27. N-(3-Pyridyl)-N'-(1,2,2-trimethylpropyl)-terephthalamid
28. N-(4-Pyridyl)-N'-(1,2,2-trimethylpropyl)-terephalamid
29. N-(3-Pyridyl)-N'-(1,2,2-trimethylpropyl)-isophthalamid
30. N-(4-Pyridyl)-N'-(1,2,2-trimethylpropyl)-isophtalamid
31. N-(6-Chlor-3-pyridyl)-N'-( " " )-phthalamid
32. N-(3-Pyridyl)-N'-( " - " )-furandicarbonamid
33. N-(4-Pyridyl)-N'-( " - " )-pyrazin-2,3-dicarbonamid
34. 3-/N-(1,2,2-Trimethylpropyl)-carbamoyl7-pyridin-2-carbonsäure-N-(3-pyridyl)-amid
35. 2-/N-(1,2,2-Trimethylpropyl)-carbamoyl7-pyridin-3-carbonsäure-N-(3-pyridyl)-amid.

Insbesondere seien genannt:

1. N-(4-Pyridyl)-N'-(1,2,2-trimethylpropyl)-thiodiglykolsäureamid
2. N-(3-Pyridyl)-N'-(1,2,2-trimethylpropyl)-phthalamid.

Die Erfindung betrifft weiterhin neue Verbindungen der Formel I, die dadurch gekennzeichnet sind, daß

$R_1$ einen eingangs beschriebenen Heteroarylrest darstellt,

A und $R_2$ die bereits erwähnte Substituentendefinition besitzt,

mit der Ausnahme, daß A keineEinfachbindung darstellt, falls $R_1$ eine Pyridylgruppe bedeutet.

Die beschriebenen Verbindungen der Formel I lassen sich beispielsweise nach folgenden Verfahrensvarianten erhalten:

A.

Die Variante A ist durch folgendes Schema gekennzeichnet:

a) $$\underset{II}{HO\text{-}CO\text{-}A\text{-}COOR_4} \longrightarrow \underset{III}{R_3\text{-}CO\text{-}A\text{-}COOR_4}$$

b) $$\underset{III}{R_3\text{-}CO\text{-}A\text{-}COOR_4} + \underset{IV}{R_2\text{-}NH_2} \longrightarrow \underset{V}{R_2\text{-}NH\text{-}CO\text{-}A\text{-}COOR_4}$$

c) $$R_2\text{-}NH\text{-}CO\text{-}A\text{-}COOR_4 \longrightarrow R_2\text{-}NH\text{-}CO\text{-}A\text{-}COOH$$

d) $$R_2\text{-}NH\text{-}CO\text{-}A\text{-}COOH \longrightarrow R_2\text{-}NH\text{-}CO\text{-}A\text{-}CO\text{-}R_3$$

e) $$\underset{VII}{R_2\text{-}NH\text{-}CO\text{-}A\text{-}CO\text{-}R_3} + \underset{VIII}{R_1\text{-}NH_2} \longrightarrow \underset{I}{R_2\text{-}NH\text{-}CO\text{-}A\text{-}CO\text{-}NHR_1}$$

A, $R_1$ und $R_2$ besitzen die bereits angegebenen Bedeutungen.

$R_3$ steht für einen elektronenziehenden Rest, wie er in der Peptidchemie zur Aktivierung einer Carbonsäuregruppierung üblich ist (Houben-Weyl 15/2 (1974); Schröder, Lübke, The Peptides Vol. 1 (1965)).

$R_4$ steht für einen geraden oder verzweigten Alkylrest, bevorzugt mit 1 bis 4 Kohlenstoffatomen, der gegebenenfalls 1-3 Reste aus der Gruppe Chlor, Methoxy, Cyano, Nitro, Phenyl, Carboxy, Carboxymethyl, 4-Nitrophenyl trägt, insbesondere Methyl, Ethyl, Propyl, t-Butyl, 2,2,-Trichlorethyl, Methoxymethyl, Cyanomethyl, Nitromethyl, Phenylmethyl, Diphenylmethyl, Carboxymethyl, Methylcarboxymethyl, 4-Nitrophenylmethyl, 2-Cyanoethyl, 2-Chlorethyl, 2-Nitroethyl.

Im Verfahrensschritt ⓑ kann statt eines Amins der allgemeinen Formel (IV) auch ein Amin der allgemeinen Formel (VIII) eingesetzt werden, wenn im Verfahrensschritt ⓔ die Amine (IV) und (VIII) ebenfalls vertauscht werden.

In der Verfahrensvariante A wird im Schritt ⓐ die Aktivierung der Carbonsäure (II) zu einer Verbindung der allgemeinen Formel (III) nach in der Literatur (Houben-Weyl 15/2 (1974)) beschriebenen Verfahren vorgenommen.

Im Verfahrensschritt ⓑ wird dieses reaktive Carbonsäurederivat (III) mit einem Amin der allgemeinen Formel (IV) umgesetzt bei bevorzugt -70°C bis 40°C, insbesondere -70°C bis 5°C. Die Umsetzung kann in Gegenwart einer Base wie Triethylamin, Ethyldiisopropylamin, N-Methylmorpholin, Pyridin, 4-Dimethylaminopyridin, N,N-Dimethylanilin erfolgen.

Als Lösungsmittel kommen in Frage: chlorierte Kohlenwasserstoffe, z.B. Dichlormethan, Chloroform, 1,2-Dichlorethan, Ether, z.B. Diethylether, Tetrahydrofuran, Dioxan, 1,2-Dimethoxyethan, aromat. Kohlenwasserstoffe, z.B. Benzol oder Toluol, Acetonitril, Nitromethan, DMF, Hexamethylphosphorsäuretriamid, Pyridin, Essigester, Aceton.

Die Umsetzung kann unter Inertgasatmosphäre, z.B. Stickstoff, Kohlendioxid, Argon, durchgeführt werden, wird im allgemeinen aber unter Luft durchgeführt.

Die Umsetzung kann bei Normaldruck, aber auch bei erhöhtem Druck durchgeführt werden. Im allgemeinen arbeitet man bei Normaldruck.

Bei dieser Umsetzung kann das Mol-Verhältnis des Amins IV oder VIII zu dem aktivierten Carbonsäurederivat (III) in einem Bereich von 10:1 bis 0,1:1 variiert werden. Vorzugsweise arbeitet man in einem Bereich, der zwischen 2:1 und 0,5:1 liegt.

Im Verfahrensschritt (c) wird die Verbindung der allgemeinen Formel (V) bzw. (Va) in eine Carbonsäure der allgemeinen Formel (VI) bzw. (VIa) umgewandelt. Dabei wird die Verbindung (V) bzw. (Va) hydrolysiert. Die Hydrolyse wird in einem alkolischen Lösungsmittel, wie Methanol oder Ethanol, oder in einen Gemisch von Wasser mit einem inerten organischen Lösungsmittel wie Methanol, Ethanol, Dioxan, Tetrahydrofuran unter Erhitzen auf den Siedepunkt des Lösungsmittels oder Lösungsmittelgemischs in Gegenwart einer Base, beispielsweise ein Alkalimetall- oder Erdalkalimetallhydroxid, wie Natrium- oder Magnesiumhydroxid, ein Alkalimetallcarbonat, wie Natrium- oder Kaliumcarbonat, oder ein Alkalimetallalkoxid, wie Natriummethylat oder Kalium-tert.-butylat, durchgeführt.

Im Verfahrensschritt ⓓ erfolgt die Aktivierung der im Verfahrensschritt ⓒ erhaltenen Verbindungen (VI) bzw. (VIa) zu einer Verbindung (VII) bzw. (VIIa) nach in der Literatur beschriebenen Verfahren (Houben-Weyl 15/2 (1974) und darin zitierte Literaturstellen).

Die erhaltene Verbindung (VII) bzw. (VIIa) wird mit einem Amin der allgemeinen Formel (VIII) oder (IV) bei -70°C bis 40°C, insbesondere bei -60°C bis 10°C umgesetzt. Die Umsetzung kann in Gegenwart einer Base wie Triethylamin, Ethyldiisopropylamin, N-Methylmorpholin, Pyridin, 4-Dimethylaminopyridin oder N,N-Dimethylanilin erfolgen. Als Lösungsmittel für die Umsetzung kommen in Frage:

chlorierte Kohlenwasserstoffe, z.B. Dichlormethan, Chloroform, 1,2-Dichlorethan,

Ether, z.B. Diethylether, Tetrahydrofuran, Dioxan, 1,2-Dimethoxyethan,

aromat. Kohlenwasserstoffe, z.B. Benzol, Toluol, Acetonitril, Nitromethan, Dimethylformamid, Hexamethylphosphorsäuretriamid, Pyridin, Essigsäureethylester, Aceton.

Die Umsetzung kann unter Normaldruck, aber auch bei erhöhtem Druck durchgeführt werden. Im allgemeinen arbeitet man bei Normaldruck.

Bei dieser Umsetzung kann das Mol-Verhältnis des Amins VIII zu den reaktiven Carbonsäurederivat VII in einem Bereich von 10:1 bis 0,1:1 variiert werden. Vorzugsweise arbeitet man in einem Bereich, der zwischen 2:1 und 0,5:1 liegt.

Die Verbindungen der allgemeinen Formeln (III) und (VII) gehören, je nach der Bedeutung des Restes $R_3$, folgenden Substanzklassen an und sind bekannt bzw. können nach bekannten Verfahren hergestellt werden:

Ethoxycarbonyl- und Diethoxycarbonyl-methylester, 2-Oxopropylester, 2-Diethylaminoethylester, Brommethylester, Cyanmethylester, Aminocarbonyl-methylester, Propargylester, Glykolsäureester, Ribosylester, Phenylester, Nitrophenylester, Dinitrophenylester, Dichlor-nitrophenylester, Trichlorphenylester, Pentachlorphenylester, Pentafluorphenylester, 4-Methylsulfonylphenylester, Phenylazophenylester, 4-Cyanphenylester, Chinolyl-(8)-ester, 1-Ethoxycarbonyl-2-ethoxy-1,2-dihydro-chinolylester, Pyridyl-(3)-ester, 2-Hydroxyphenylester, 4-Nitro-guaiacylester, 4-Dimethylaminophenylester, 4-Aminosulfonylphenylester, 4-(Acetylamino-sulfonyl)-phenylester, 4-Propionyl-phenylester, Vinylester, 1-Methyl-2-acetylvinylester, 2,2-Diphenylvinylester, 2-Cyan-2-phenylvinylester, Methoxy-methylester, Tetrahydropyranyl-(2)-ester, 1-Methoxy-vinylester, 1-Ethoxy-vinylester, Dimethylaminovinylester, N,N'-Dicyclohexyllactimester, N-Ethyl-N'-(3-dimethylaminopropyl)-lactimester, 2-Hydroxypyridylester, O-Acyl-N,N-dimethyl-hydroxylamine, O-Acyl-N,N-diethyl-hydroxylamine, O-Acyl-N,N-dibenzyl-hydroxylamine, (N-Hydroxy-piperidin)-ester, O-Acyl-N-isopropyliden-hydroxylamine, (N-Hydroxy-pivaloamid)-ester, (N-Hydroxybenzamid)-ester, (1,2-Dihydro-pyridonyl-(1))-ester, (N-Hydroxy-succinimid)-ester, (N-Hydroxyglutarimid)-ester, (N-Hydroxy-phthalimid)-ester, (N-Hydroxychinolinsäureimid)-ester, O-Methyl-Kohlensäureanhydride, O-Ethyl-Kohlensäureanhydride, O-Isobutyl-Kohlensäureanhydride, O-Benzyl-Kohlensäureanhydride, O-Phenyl-Kohlensäureanhydride, 2-Ethylbuttersäureanhydride, 2,2-Dimethyl-propionsäureanhydride, Diphenylessigsäureanhydride, Benzoesäureanhydride, 4-Methoxybenzoesäureanhydride, O,O-Dibenzylphosphorsäureanhydride, O,O-Di-(4-nitrobenzyl)-phosphorsäureanhydride, Methansulfonsäureanhydride, Benzolsulfonsäureanhydride, 4-Methylbenzolsulfonsäureanhydride, Trifluormethylsulfonsäureanhydride, Nonafluorbutylsulfonsäureanhydride, Phenylthioester, 4-Nitrophenylthioester, Phenylselenoester, Carbonsäureazide, Carbonsäureimidazolide, Carbonsäure-1,2,4-triazolide, Carbonsäure-1,2,4-oxadiazolinone-(5), Carbonsäurechloride, Carbonsäurebromide, Carbonsäurejodide, Carbonsäurecyanide.

Die Amine der Formeln IV und VIII sind ebenfalls bekannt.

Namentlich seien folgende Amine der Formel IV angegeben:

t-Butylamin, 1,1-Dimethylpropylamin, 2,2-Dimethylpropylamin, 1,2,2-Trimethylpropylamin, 1,1-Dimethylbutylamin

1,1,2-Trimethylpropylamin, 1-Ethyl-1-methylpropylamin, 1,1,3-Trimethylbutylamin, 1,1-Diethylpropylamin, 1,1-Diethylbutylamin, 1,1,2-Triethylbutylamin, 1,1,2,2-Tetramethylpropylamin.

Namentlich seien folgende Amine der Formel VIII aufgeführt:

Anilin, Naphthylamin, Amino-(n-butyl)benzol, Amino-(methoxy)benzol, Amino-(ethoxy)benzol, Amino-(n-butoxy)benzol, Amino-(methylthio)benzol, Amino-(n-butylthio)benzol, Amino-(dimethylamino)benzol, Amino-(dibutylamino)benzol, Amino-(methylamino)benzol, Amino-(n-butylamino)benzol, Amino-chlorbenzol, Amino-brombenzol, Amino-fluorbenzol, Amino-(chlor)-(methyl)benzol, Amino-dimethylbenzol, Amino-(hydroxy)-(methyl)benzol, Amino-(mercapto)-(methyl)-benzol, Amino-trimethylbenzol, Amino-chlor-methoxybenzol, Amino-dichlorbenzol, Aminotrichlorbenzol, Amino-chlor-nitrobenzol, Amino-methyl-nitrobenzol, Amino-chlor-(trifluormethyl)benzol, Amino-bis(trifluormethyl)benzol, Amino-ethyl-methylbenzol, Amino-dichlor-methoxybenzol, Amino-chlor-(methylthio)benzol, Amino-hydroxy-(methyl)-benzol, Amino-chlor-hydroxybenzol, Amino-dimethoxybenzol, Amino-diethoxybenzol, Amino-chlor-dimethoxybenzol.

B. Die Verfahrensvariante B ist durch folgendes Schema gekennzeichnet:

a) 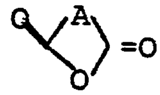 + $R_2$-$NH_2$ → $R_2$NH-CO-A-COOH

IX IV VI

b) $R_2$-NH-CO-A-COOH → $R_2$-NH-CO-A-CO-$R_3$

VI VIII

c) $R_2$-NH-CO-A-CO-$R_3$ + $R_1$-$NH_2$ → $R_2$-NH-CO-A-CO-NH-$R_1$

VII VIII I

Die Verfahrensschritte (b) und (c) der Variante B entsprechen den Schritten (d) und (e) der Variante A.

Im Verfahrensschritt (a) wird ein cyclisches Anhydrid der allgemeinen Formel (IX) mit einem Amin der allgemeinen Formel (IV) oder (VIII) bei -20°C bis + 60°C, insbesondere bei 0°C bis 30°C, in einem inerten Lösungsmittel wie einem chlorierten Kohlenwasserstoff, z.B. Dichlormethan, Chloroform, 1,2-Dichlorethan, einen Ether, z.B. Diethylether, Tetrahydrofuran, Dioxan, 1,2-Dimethoxyethan, einen aromatischen Kohlenwasserstoff, z.B. Benzol oder Toluol oder in Acetonitril, Nitromethan, Dimethylformamid, Hexamethylphosphorsäuretriamid, Pyridin, Essigsäureethylester, Aceton umgesetzt.

Die Umsetzung kann in Gegenwart einer Base wie Triethylamin, Ethyldiisopropylamin, N-Methylmorpholin, Pyridin, 4-Dimethylaminopyridin oder N,N-Dimethylanilin erfolgen.

Die Umsetzung kann bei Normaldruck, aber auch bei erhöhtem Druck durchgeführt werden. Im allgemeinen arbeitet man bei Normaldruck.

Die Amine (IV) und (VIII) können im Verfahrensschema miteinander vertauscht werden.

Bei dieser Umsetzung kann das Mol-Verhältnis des Amins IV oder VIII zu dem cyclischen Carbonsäureanhydrid IX in einem Bereich von 10:1 bis 0,1:1 variiert werden. Vorzugsweise arbeitet man in einem Bereich, der zwischen 2:1 und 0,5:1 liegt.

Die als Ausgangsverbindungen eingesetzten Amine, Dicarbonsäuren und Dicarbonsäureanhydride sind bekannt und/oder leicht herstellbar (Houben-Weyl 8 (1952); Sandler, Karo, Organic Functional, Group Preparations, Vol. I und III (1972); Patei, The Chemistry of Functional Groups, Supplement B: The chemistry of acid derivatives, Part. 1 (1979); Anschutz, Biernaux, Liebigs Annalen 273, 68; Wegscheider, Perndanner, Anspitzer, Monatshefte für Chemie 31, 1258; Cohen, De Pennigton, J. Chem. Soc. 113, 63).

Die Verbindungen der Formel I haben ein breites und vielseitiges pharmakologisches Wirkungsspektrum und überraschend lange Wirkungsdauer, sie sind daher zur Bekämpfung von Erkrankungen sehr gut geeignet.

Im einzelnen konnten im Tierexperiment folgende Hauptwirkungen nachgewiesen werden:

1. Der Tonus der glatten Muskulatur der Gefäße wird unter der Wirkung der Verbindungen stark vermindert. Diese gefäßspasmolytische Wirkung kann im gesamten Gefäßsystem stattfinden, oder sich mehr oder weniger isoliert in umschriebenen Gefäßgebieten (wie z.B. dem Zentralnervensystem) manifestieren. Die Verbindungen eignen sich daher besonders als Cerebraltherapeutika.

2. Die Verbindungen senken den Blutdruck von normotonen und hypertonen Tieren und können somit als antihypertensive Mittel verwendet werden.

3. Einige Verbindungen erhöhen den Blutdruck.

Die erfindungsgemäßen Verbindungen eignen sich aufgrund dieser Eigenschaften zur Prophylaxe der akuten und chronischen ischämischen Herzkrankheit im weitesten Sinne, zur Therapie des Hoch- bzw. Niederdrucks sowie zur Behandlung von cerebralen und peripheren Durchblutungsstörungen.

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht-toxischer pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmiteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z.B. Erdölfraktionen), pflanzliche Öle (z.B. Erdnuß-(Sesam-Öl), Alkohole (z.B. Ethylalkohol, Glycerin), Glykole (z.B.Propylenglykol, Polyethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin- Sulfitablaugen, Methylcellulose, Stärke und Poylvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung könne Tabletten selbstverständlich außer den genannten Trägerstoffen auch Zusätze, wie Natriumcitrat, Calciumcarbonat und Dicalciumphosphat zusammen mit verschiedenen Zuschlagstoffen,

wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer mit den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 10 mg/kg, vorzugsweise etwa 0,05 bis 5 mg/kg Körpergewicht pro Tag zur Erzielung wirksamer Ergebnisse zu verabreichen, und bei oraler Applikation beträgt die Dosierung etwa 0,05 bis 20 mg/kg, vorzugsweise 0,5 bis 5 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und der individuellem Verhalten gegenüber dem Medikament bzw. der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

Folgende Beispiele erläutern die Erfindung:

Beispiel 1 (Variante B)

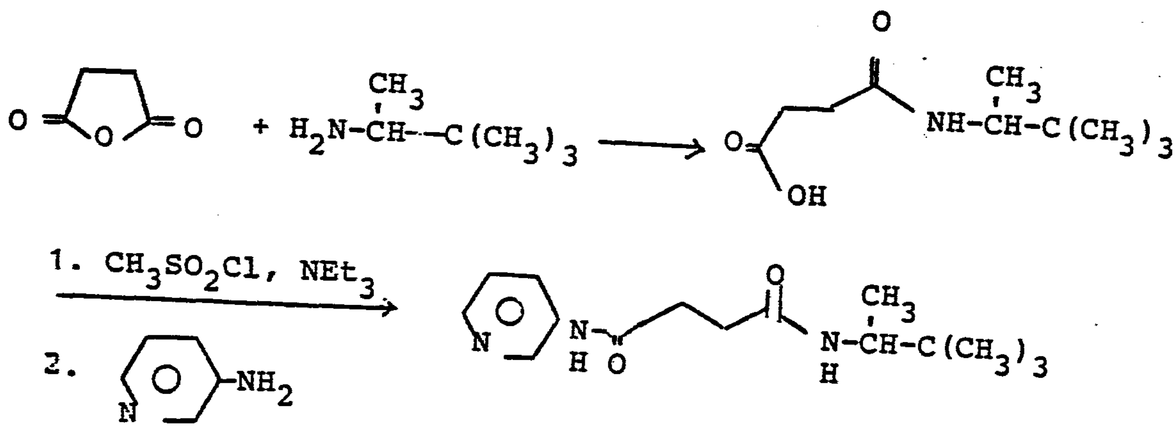


N-(3-Pyridyl)-N'-(1,2,2-trimethylpropyl)-succinamid

10 g Bernsteinsäureanhydrid werden in 150 ml Essigester gelöst und mit 10 g 3,3-Dimethyl-2-butylamin in 150 ml Essigester versetzt. Nach 2-stündigem Rühren bei Raumtemperatur wird das Lösungsmittel im Vakuum abgedampft. Man erhält so 20 g Bernsteinsäure mono (1,2,2-trimethylpropyl)amin als Öl. Dieses wird in 100 ml Methylenchlorid gelöst, mit 20 g Triethylamin versetzt und auf -50°C gekühlt. Bei dieser Temperatur werden 10 g Methansulfonsäurechlorid in 10 ml Methylenchlorid zugetropft. Man läßt 30 Min. bei -50°C nachrühren und gibt dann 9,4 g 3-Aminopyridin in 30 ml Methylenchlorid zu dem Reaktionsgemisch. Der Ansatz wird noch 16 h bei Raumtemperatur gerührt. Dann wird das Reaktionsgemisch 3 x

mit Methylenchlorid extrahiert. Die vereinigten organischen Phasen werden mit Natriumsulfat getrocknet und eingedampft. Der Rückstand wird an Kieselgel mit Methylenchlorid/Methanol 95/5 als Laufmittel chromatographiert. Die produkthaltigen Fraktionen kristallisieren nach dem Eindampfen aus. Nach Aufschlämmen in Ether werden die Kristalle abgesaugt. Man erhält so 15 g (54 % der theor. Ausbeute) der Titelverbindung, die einen Schmelzpunkt von 130-133°C aufweist.

Beispiel 2 (Variante A)

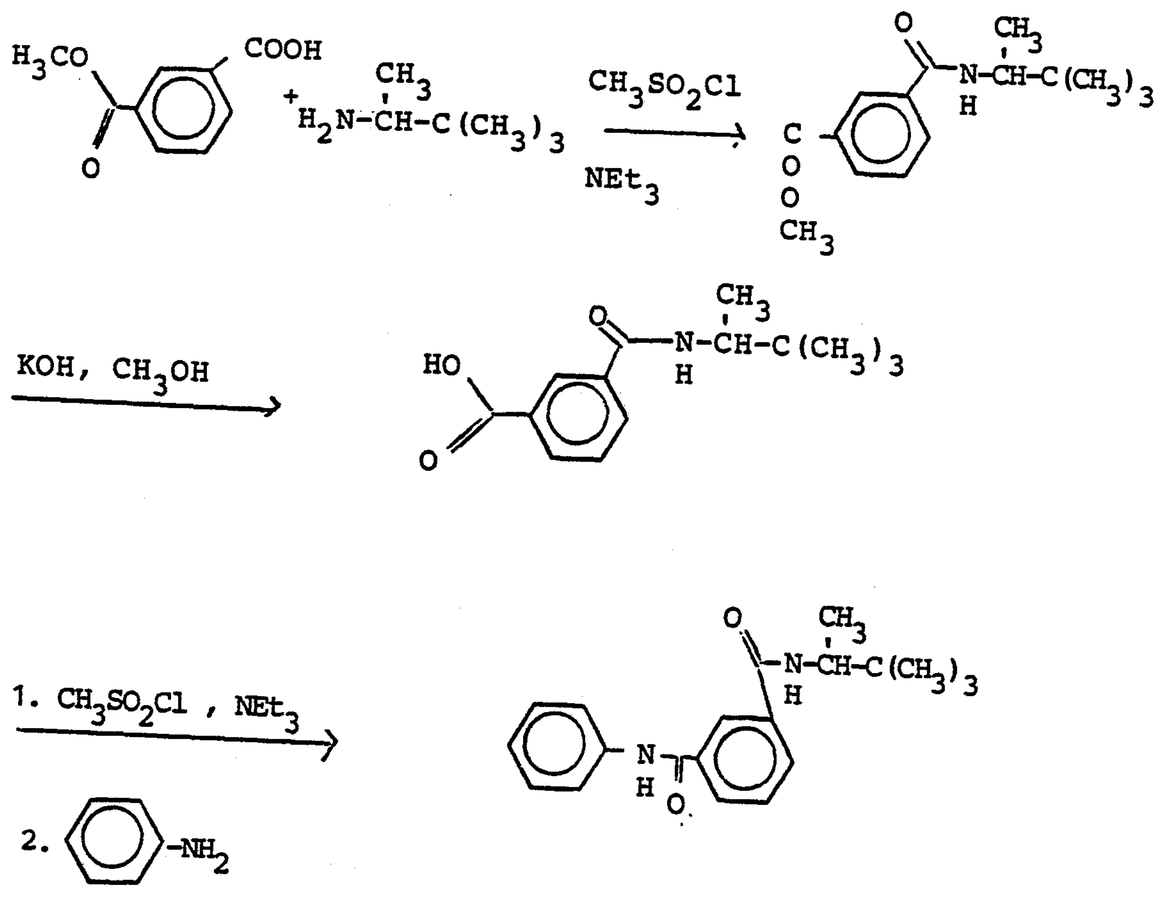

N-Phenyl-N'-(1,2,2-trimethylpropyl)-isophthalamid

16,8 g Isophthalsäuremonomethylester werden in 200 ml Methylenchlorid gelöst und mit 20 g Triethylamin versetzt. Bei -50°C werden dann 10 g Methansulfonsäurechlorid in 10 ml Methylenchlorid zugetropft. Man läßt 30 Min. bei -50°C nachrühren und gibt dann 10 g 3,3-Dimethyl-2-butylamin in 20 ml Methylenchlorid zu. Nach 16 stündigem Rühren bei Raumtemperatur wird 3 x mit 2n Schwefelsäure und 3 x mit Natriumhydrogencarbonatlösung extrahiert. Die organische Phase wird mit Natriumsulfat getrocknet und eingedampft. Man erhält so 19,5 g schwach gelbes, öliges Isophthalsäuremethylester-(1,2,2-trimethylpropyl)amid, die in 200 ml Ethanol gelöst und mit 40 ml 45 % NaOH versetzt werden. Das Reaktionsgemisch wird 10 h bei RT stehen gelassen, dann mit 1 l Wasser verdünnt und 2 x mit Methylenchlorid extrahiert. Die wäßrige Phase wird mit 2n Schwefelsäure angesäuert und 3 x mit Methylenchlorid extrahiert. Letztere organische Phasen werden vereinigt und mit Natriumsulfat getrocknet. Nach Eindampfen erhält man 12,9 g weißes, kristallines Isophtalsäuremono-(1,2,2-trimethylpropyl)amid vom Schmelzpunkt 210-212°C. 12,9 g dieser Säure werden in 100 ml Methylenchlorid gelöst und mit 10,5 g Triethylamin versetzt. Bei -50°C werden 5,4 g Methansulfonsäurechlorid in 10 ml Methylenchlorid zugetropft. Man läßt 30 Min. bei -50°C nachrühren und gibt dann 4,8 g Anilin in 5 ml Methylenchlorid zu. Man läßt 16 h bei Raumtemperatur rühren und extrahiert die Reaktionslösung dann 2 x mit 2 n Schwefelsäure. Die organische Phase wird mit Natriumsulfat getrocknet und

eingedampft. Der kristalline Rückstand wird in Ether aufgeschlämmt und abgesaugt. Man erhält so 12,5 g (74 % der theoretischen Ausbeute) der Titelverbindung, die einen Schmelzpunkt von 216-218°C aufweist.

Beispiele 3 bis 35

Die Herstellung weiterer Verbindungen der allgemeinen Formel I ist in der nachfolgenden Tabelle 1 erläutert. In der Spalte "Methode" ist die Art der Umsetzung angegeben, nach der die Produkte der jeweiligen Beispiele hergestellt werden; vgl. die entsprechenden Methoden in den vorstehenden Beispielen.

Tabelle 1

$R_1-\underset{H}{N}-\underset{\overset{\|}{O}}{C}-A-\underset{\overset{\|}{O}}{C}-\underset{H}{N}-R_2$

| Beispiel-Nr. | A | $R_1$ | $R_2$ | Methode | Ausbeute [%] | Fp [°C] |
|---|---|---|---|---|---|---|
| 3 | Einfach-bindung | 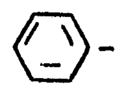 | $-\overset{CH_3}{\overset{|}{CH}}-C(CH_3)_3$ | B | 17 | 116-118 |
| 4 | Einfach-bindung | 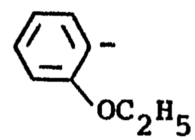 | " | B | 21 | 96- 98 |
| 5 | 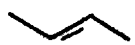 | 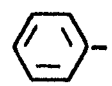 | " | B | 42 | 205-210 |
| 6 | 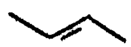 | 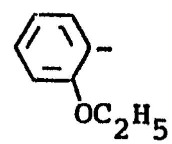 | " | B | 35 | 242-244 |
| 7 | $-CH_2-CH_2-$ | 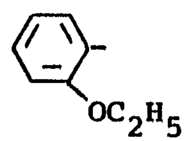 | " | A | 56 | 183-139 |
| 8 | $-CH_2-CH_2-$ | 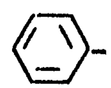 | " | A | 60 | 152-153 |

Tabelle 1 (Fortsetzung)

| Beispiel Nr. | A | $R_1$ | $R_2$ | Methode | Ausbeute [%] | Fp [°C] |
|---|---|---|---|---|---|---|
| 9 | $-CH_2-CH_2-$ | 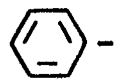 | $-CH_2-C(CH_3)_3$ | A | 38 | 162 |
| 10 | $-CH_2-S-CH_2-$ | 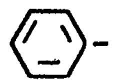 | $-\overset{CH_3}{CH}-C(CH_3)_3$ | A | 38 | 88-90 |
| 11 | $-CH_2-S-CH_2-$ | 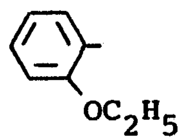 | " | A | 42 | 78 |
| 12 | 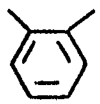 | 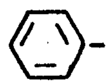 | " | A | 43 | 210 |
| 13 | 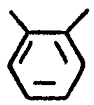 | 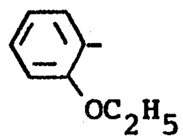 | " | A | 32 | 125 |
| 14 | 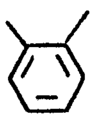 | 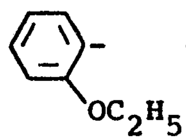 | $-CH_2-C(CH_3)_3$ | A | 46 | 155-157 |

Tabelle 1 (Fortsetzung)

| Beispiel Nr. | A | $R_1$ | $R_2$ | Methode | Ausbeute [%] | Fp [°C] |
|---|---|---|---|---|---|---|
| 15 | 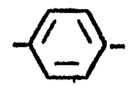 | 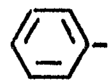 | $CH_3$<br>$-CH-C(CH_3)_3$ | B | 9 | 225-230 |
| 16 | 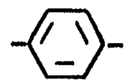 | 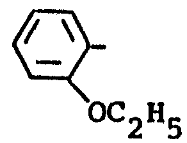 | " | B | 41 | 168-169 |
| 17 | 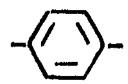 | 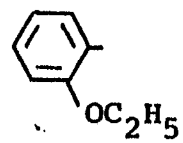 | $-CH_2C(CH_3)_3$ | B | 56 | 170-173 |
| 18 | 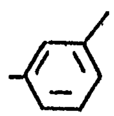 | 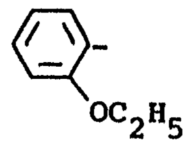 | $CH_3$<br>$-CH-C(CH_3)_3$ | B | 63 | 145-147 |
| 19 | Einfach-<br>bindung | 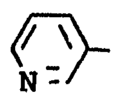 | $CH_3$<br>$-CH-C(CH_3)_3$ | B | 20 | 133,-135 |
| 20 | Einfach-<br>bindung | 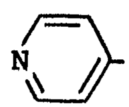 | " | B | 14 | 132-135 |

**Tabelle 1** (Fortsetzung)

| Beispiel-Nr. | A | $R_1$ | $R^2$ | Methode | Ausbeute [%] | Fp [°C] |
|---|---|---|---|---|---|---|
| 21 | 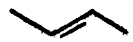 | 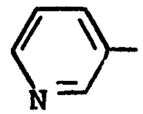 | $-\overset{CH_3}{\overset{\mid}{C}H}-C(CH_3)_3$ | B | 20 | 238-240 |
| 22 | $-CH_2-CH_2-$ | 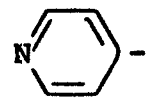 | " | A | 49 | 158-160 |
| 23 | $-CH_2-CH_2-$ | 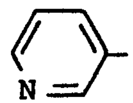 | " | A | 54 | 130-133 |
| 24 | $-CH_2-S-CH_2-$ | 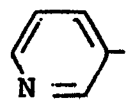 | " | A | 4 | 126-127 |
| 25 | $-CH_2-S-CH_2-$ | 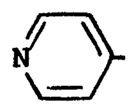 | " | A | 6,5 | 39 |
| 26 | 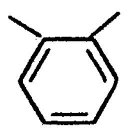 | 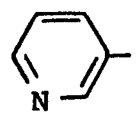 | " | A | 39 | 210-211 |

Tabelle 1 (Fortsetzung)

| Beispiel-Nr. | A | $R_1$ | $R_2$ | Methode | Ausbeute [%] | Fp [°C] |
|---|---|---|---|---|---|---|
| 27 | 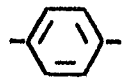 | 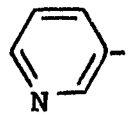 | $-CH(CH_3)-C(CH_3)_3$ | B | 40 | 254 |
| 28 | 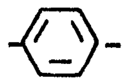 | 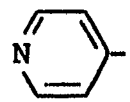 | " | B | 56 | 170-173 |
| 29 | 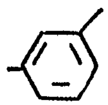 | 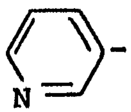 | " | B | 38 | 204-208 |
| 30 | 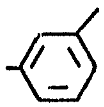 | 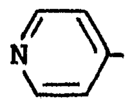 | " | B | 55 | 192-202 |
| 31 | 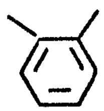 | 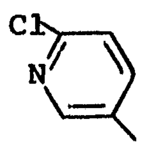 | " | B | 31 | 232-233 |
| 33 | 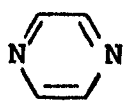 | 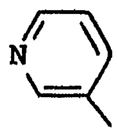 | " | B | 46 | 96 |

Tabelle 1 (Fortsetzung)

| Beispiel-Nr. | A | $R_1$ | $R_2$ | Methode | Ausbeute [%] | Fp [°C] |
|---|---|---|---|---|---|---|
| 34 | 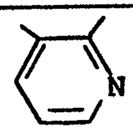 | 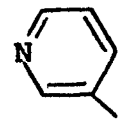 | $-\overset{CH_3}{CH}-C(CH_3)_3$ | B | 5 | 197-199 |
| 35 | 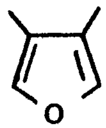 | 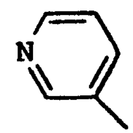 | " | B | 51 | 62-66 |

Die in der folgenden Tabelle angeführten Verbindungen senken an der spontan hypertensiven Ratte den Blutdruck um mindestens 15 mm Hg. Angegeben ist die niederste noch wirksame, orale Dosis.

| Beispiel-Nr. | Dosis [mg/kg KG p.o.] |
|---|---|
| 12 | 10 |
| 13 | 10 |
| 26 | 3 |

## Patentansprüche

1. Bis-(carboxamid)-Verbindungen der Formel I

$$R_1-\underset{H}{N}-\underset{\overset{\|}{O}}{C}-A-\underset{\overset{\|}{O}}{C}-\underset{H}{N}-R_2$$

in der

$R_1$ einen Arylrest, der gegebenenfalls substituiert ist durch gleiche oder verschiedene Substituenten aus der Gruppe Halogen, $C_1$-$C_4$-Alkyl, $C_1$-$C_4$-Alkoxy, $C_1$-$C_4$-Alkylmercapto, jeweils $C_1$-$C_4$-Dialkylamino, $C_1$-$C_4$-Monoalkylamino, Hydroxy, Mercapto, Amino, Nitro, Cyano, Trifluormethyl oder einen Heteroarylrest mit 5-6 Ringatomen, wobei 1 oder 2 Atome Stickstoff, Sauerstoff oder Schwefel sind, der gegebenenfalls einfach substituiert ist durch Substituenten aus der Gruppe Halogen, Trifluormethyl, Nitro, Cyano, $C_1$-$C_4$-Alkyl, $C_1$-$C_4$-Alkoxy, $C_1$-$C_4$-Alkylmercapto darstellt,

$R_2$ einen verzweigten Alkylrest oder einen Cycloalkylrest bedeutet,

A eine Einfachbindung, einen Alkylenrest, einen Alkenylrest, eine Gruppe der Formel $-CH_2-S-CH_2-$ oder $-CH_2-O-CH_2-$, einen bivalenten Cycloalkylenrest, einen bivalenten

Heteroarylrest mit 5-6 Ringatomen, wobei 1 oder 2 Atome Stickstoff, Sauerstoff oder Schwefel sind, darstellt,

sowie ihre physiologisch unbedenklichen Salze, zur Bekämpfung von Erkrankungen.

2. Bis-(carboxamid)-Verbindungen gemäß Formel 1 in Anspruch 1, in der

$R_1$ einen Phenylrest darstellt, der gegebenenfalls 1-3 mal substituiert ist durch gleiche oder verschiedene Substituenten aus der Gruppe Halogen, Trifluormethyl, Nitro, Cyano, Hydroxy, Amino, $C_1$-$C_4$-Alkyl, $C_1$-$C_4$-Alkoxy, $C_1$-$C_4$-Alkylmercapto, jeweils $C_1$-$C_4$-Dialkylamino, oder einen Heteroarylrest mit 5-6 Ringatomen darstellt, wobei 1 oder 2 Atome Stickstoff, Sauerstoff oder Schwefel sind, der gegebenenfalls einfach substituiert ist durch Substituenten aus der Gruppe Halogen, Trifluormethyl, Nitro, Cyano, $C_1$-$C_4$-Alkyl, $C_1$-$C_4$-Alkoxy, $C_1$-$C_4$-Alkylmercapto,

$R_2$ einen verzweigten $C_4$-$C_{10}$-Alkylrest oder einen $C_6$-$C_{10}$-Cycloalkylrest darstellt,

A eine Einfachbindung, einen $C_1$-$C_5$-Alkylenrest, einen $C_2$-$C_6$-Alkenylrest, einen bivalenten $C_4$-$C_6$-Cycloalkylenrest, eine Gruppe

der Formel $-CH_2-S-CH_3-$ oder $-CH_2-O-CH_2-$, einen bivalenten $C_6-C_7$-Cycloalkenylrest, einen $C_6-C_{10}$-Arylenrest oder einen Heteroarylrest aus der Gruppe Furan, Thiophen, Imidazol, Oxazol, Thiazol, Pyrazol, Pyrazin oder Pridin, darstellt,

sowie ihre physiologisch unbedenklichen Salze zur Bekämpfung von Erkrankungen.

3. Bis-(carboxamid)verbindungen gemäß Formel I in Anspruch 1, in der

$R_1$ einen Phenylenrest darstellt, der gegebenenfalls 1-3 mal substituiert ist durch gleich oder verschiedene Substituenten aus der Gruppe Halogen, Trifluormethyl, $C_1-C_4$-Alkyl, $C_1-C_4$-Alkoxy, $C_1-C_4$-Alkylmercapto, oder Pyridyl oder Pyrimidyl darstellt, welche gegebenenfalls einfach substituiert sind durch Substituenten aus der Gruppe Halogen, Trifluormethyl, $C_1-C_4$-Alkyl, $C_1-C_4$-Alkoxy, $C_1-C_4$-Alkylmercapto,

$R_2$ einen verzweigten $C_4-C_{10}$-Alkylrest darstellt,

A eine Einfachbindung einen $C_2-C_4$-Alkylen oder Alkenylrest darstellt oder eine Gruppe der Formel $-CH_2-S-CH_2-$ oder $-CH_2-O-CH_2-$

bedeutet, oder eine Phenylengruppe oder einen bivalenten Heteroarylrest aus der Gruppe Furan, Thiophen, Imidazol, Pyrazin oder Pyridin darstellt,

sowie ihre physiologisch unbedenklichen Salze, zur Bekämpfung von Erkrankungen.

4. Bis-(carboxamid)verbindungen gemäß Formel I in Anspruch 1, in der

$R_1$ Phenyl, 4-Hydroxyphenyl, 2-Ethoxyphenyl, Pyridyl, 6-Chlorpyridyl darstellt,

$R_2$ tert.-Butyl, 2,2-Dimethylpropyl, 1,2,2-Trimethylpropyl, 1,1,2,2-Tetramethylpropyl bedeutet,

A eine Einfachbindung, Methylen, Ethylen, Vinylen, Phenylen, die Gruppe $-CH_2-S-CH_2-$, einen bivalenten Heteroarylrest aus der Gruppe Furan, Thiophen, Pyrazin oder Pyridin darstellt,

sowie ihre pharmazeutisch unbedenklichen Salze zur Bekämpfung von Erkrankungen.

5. Bis-(carboxamid)verbindungen gemäß Ansprüchen 1-4 zur Prophylaxe der akuten und chronischen ischämischen Herzkrankheit, zur Therapie des Hoch- bwz. Niederdruckes sowie zur Behandlung von cerebralen und peripheren Durchblutungsstörungen.

6. Bis-(carboxamid)verbindungen der Formel I gemäß Ansprüche 1-4, in der

$R_1$ einen erwähnten Heteroarylrest darstellt,

A und $R_2$ die erwähnten Substituentendefinitionen besitzen,

mit der Ausnahme, daß A keine Einfachbindung darstellt, falls $R_1$ eine Pyridylgruppe bedeutet.

7. Arzneimittel enthaltend mindestens eine Verbindung der Formel (I) gemäß Ansprüchen 1-4.

8. Verfahren zur Herstellung von Arzneimitteln, dadurch gekennzeichnet, daß man Verbindungen der Formel (I) gemäß Ansprüchen 1-4 gegebenenfalls unter Verwendung von üblichen Hilfs- und Trägerstoffen in eine geeignete Applikationsform überführt.

9. Verfahren zur Herstellung der Verbindungen gemäß Ansprüchen 1-4 und 6, dadurch gekennzeichnet, daß man entweder

a) eine Verbindung der Formel II

$$HOCO\text{-}A\text{-}COOR_4 \qquad (II)$$

in bekannter Weise in eine Verbindung der Formel III

$R_3CO\text{-}A\text{-}COOR_4$ (III)

überführt, anschließend diese Verbindung III mit einem Amin der Formel IV

$R_2\text{-}NH_2$ (IV)

oder einen Amin der Formel VIII

$R_1\text{-}NH_2$ (VIII)

zu der Verbindung V bzw. Va umsetzt

$R_2NH\text{-}CO\text{-}A\text{-}COOR_4$ (V)

$R_1NH\text{-}CO\text{-}A\text{-}COOR_4$ (Va)

anschließend in bekannter Weise die Verbindungen V bzw. Va in eine Carbonsäure der Formel VI bzw.VIa umwandelt

$R_2NH\text{-}CO\text{-}A\text{-}COOH$ (VI)

$R_1NH\text{-}CO\text{-}A\text{-}COOH$ (VIa)

anschließend diese Verbindungen in bekannter Weise in die Verbindungen VII bzw. VIIa überführt

$R_2\text{-}NH\text{-}CO\text{-}A\text{-}COR_3$ (VII)

$R_1\text{-}NH\text{-}CO\text{-}A\text{-}COR_3$ (VIIa)

und anschließend diese Verbindungen VII bzw. VIIa mit einem Amin der Formel IV oder VIII umsetzt, oder

b) eine Verbindung der Formel IX

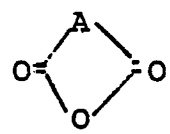

(IX)

mit einem Amin der Formel IV oder VIII zu einer Verbindung der Formel VI oder VIa umsetzt

$R_2$-NH-CO-A-COOH (VI)

$R_1$-NH-CO-A-COOH (VIa),

anschließend diese Verbindung in bekannter Weise in die Verbindungen VII bzw. VIIa überführt,

$R_2$-NH-CO-A-$COR_3$ (VII)

$R_1$-NH-CO-A-$COR_3$ (VIIa)

und anschließend diese Verbindungen VII bzw. VIIa mit einem Amin der Formel IV oder VIII umsetzt, wobei

A, $R_1$ und $R_2$ die in den Ansprüchen 1-4 und 6 angegebene Bedeutung besitzen und

$R_3$ für einen elektronenziehenden Rest steht und

$R_4$ eine Alkylgruppe darstellt, die gegebenenfalls 1-3 fach substituiert ist durch Chlor, Methoxy, Cyano, Nitro, Phenyl, Carboxy, Carboxymethyl und/oder 4-Nitrophenyl.